# EUROPEAN PATENT APPLICATION

(11) **EP 4 517 328 A1**
(43) Date of publication of application: **05.03.2025**
(21) Application number: 23796127.1
(22) Date of filing: 12.04.2023
(51) Int. Cl.: G01N 35/00, G01N 35/02

(54) **INSPECTION DEVICE**

(30) Priority: 28.04.2022 JP 2022075196
(71) Applicant: FUJIFILM Corporation, Tokyo 106-8620 (JP)
(72) Inventor: SUGAYA, Takuo, Ashigarakami-gun, Kanagawa 258-8538 (JP)
(74) Representative: Dehns Germany Partnerschaft mbB
(86) International application number: PCT/JP2023/014931
(87) International publication number: WO 2023/210383

(57) **Abstract**

Provided is an examination apparatus which examines a specimen using a cell accommodating a reagent solidified with gelatin, the examination apparatus including a heating unit for dissolving the gelatin, a camera for imaging a state of the gelatin in the cell, and a processor for controlling the heating unit and the camera, in which the processor is configured to acquire an image of the state of the gelatin, which is imaged by the camera, and analyze the image to determine a presence or absence of undissolved gelatin or a presence or absence of a sign of undissolved gelatin.

## Description

### BACKGROUND OF THE INVENTION

### 1. Field of the Invention

The present disclosure relates to an examination apparatus.

### 2. Description of the Related Art

An examination apparatus which quantitatively or qualitatively detects a target substance in a specimen has been known. Such an examination apparatus mainly utilizes an immunoassay principle, and examples thereof include a chemiluminescent enzyme immunological analysis apparatus and a fluorescence immunological analysis apparatus (for example, JP2016-085093A).

In these examination apparatuses, a configuration using a cartridge provided with a cell which accommodates a reagent used in one examination has been proposed (for example, JP1999-316226A (JP-H11-316226A)).

### SUMMARY OF THE INVENTION

In a case where the reagent to be accommodated in the cell of the cartridge is particles, a method of accommodating the reagent in a state of being enclosed in gelatin has been studied in order to prevent the reagent from scattering in the cell during transportation or the like. In this case, in the examination, the cartridge is loaded into the examination apparatus, and the gelatin is dissolved by heating in the apparatus to separate the gelatin and the reagent, and then the reagent and the specimen are mixed.

In such a cartridge, the gelatin in the cell may be dissolved due to a temperature environment, a vibration, or the like during the transportation or the like, and the gelatin may be in a non-uniform state, such as a state in which a part of the gelatin is attached to a part of a wall surface of the cell. In a case where a part of the gelatin in the cell is in a state of being attached to the wall surface of the cell, the gelatin may not be dissolved while being attached to the wall surface during the dissolution of the gelatin, and thus the gelatin may remain. In a case where the gelatin remains as it is and the mixing with the specimen is carried out, an adverse effect such as defective mixing occurs. It is desired to suppress the poor solubility of the gelatin, but since the gelatin is dissolved inside the apparatus, it is difficult to grasp the presence or absence of the poor solubility of the gelatin in the cell and the presence or absence of a sign of the poor solubility.

An object of the present disclosure is to provide an examination apparatus capable of grasping the presence or absence of undissolved gelatin or the presence or absence of a sign of undissolved gelatin in a cell accommodating a reagent in a state of being enclosed in the gelatin.

An examination apparatus according to the present disclosure examines a specimen using a cell accommodating a reagent solidified with gelatin, the examination apparatus including a heating unit for dissolving the gelatin, a camera for imaging a state of the gelatin in the cell, and a processor for controlling the heating unit and the camera, in which the processor is configured to acquire an image of the state of the gelatin, which is imaged by the camera, and analyze the image to determine a presence or absence of undissolved gelatin or a presence or absence of a sign of undissolved gelatin.

The processor may be configured to dissolve the gelatin by the heating unit under a preset heating condition, configured to image the state of the gelatin by controlling the camera, configured to derive a dissolved state of the gelatin after dissolving the gelatin, and configured to determine the presence or absence of the undissolved gelatin.

In a case where it is determined that the gelatin is undissolved or there is a sign of the undissolved gelatin, the processor may be configured to promote dissolution of the gelatin by controlling the heating unit.

The processor may be configured to perform at least one of control of extending a dissolving time of the gelatin or control of increasing a dissolving temperature of the gelatin with respect to the heating unit.

A notification unit for notifying an error may be further provided, in which, in a case where it is determined that the gelatin is undissolved or there is a sign of the undissolved gelatin, the processor may be configured to notify the undissolved gelatin or the sign of the undissolved gelatin by the notification unit.

In the examination apparatus according to the present disclosure, an examination target substance in the specimen may be detected by an antigen-antibody reaction.

The reagent may be magnetic particles modified with a binding substance specifically bound to the examination target substance.

According to the technology of the present disclosure, it is possible to grasp the presence or absence of undissolved gelatin in a cell accommodating a reagent in a state of being enclosed in gelatin.

### BRIEF DESCRIPTION OF THE DRAWINGS

Fig. 1 is a schematic view showing a configuration of an examination apparatus according to an embodiment.
Fig. 2A is a top view of a cartridge, and Fig. 2B is a front view thereof.
Fig. 3 is a diagram showing an examination step.
Fig. 4 is a diagram showing a positional relationship of a camera and a reaction cell.
Fig. 5 is a schematic diagram of an image imaged after dissolving gelatin.
Fig. 6 is a diagram showing a first flow of a gelatin dissolution and removal process.
Fig. 7 is a diagram showing a step from discharging the gelatin in a uniform state to a first reaction.
Fig. 8 is a diagram showing a step from discharging the gelatin in a non-uniform state to a first reaction.
Fig. 9 is a schematic diagram of an image imaged before dissolving gelatin.
Fig. 10 is a diagram showing a state before and after dissolution of the gelatin in a case where the gelatin is uniformly accommodated.
Fig. 11 is a diagram showing a state before and after dissolution of the gelatin in a case where the gelatin is non-uniformly accommodated.
Fig. 12 is a diagram showing a second flow of the gelatin dissolution and removal process.
Fig. 13 is a diagram showing a third flow of the gelatin dissolution and removal process.
Fig. 14 is a diagram showing a fourth flow of the gelatin dissolution and removal process.

### DESCRIPTION OF THE PREFERRED EMBODIMENTS

Hereinafter, an examination apparatus according to the embodiment of the present disclosure will be described with reference to the drawings. Constituent elements indicated by the same reference numeral in the drawings mean the same constituent element. However, unless otherwise specified in the specification, each constituent element is not limited to one and may be plural.

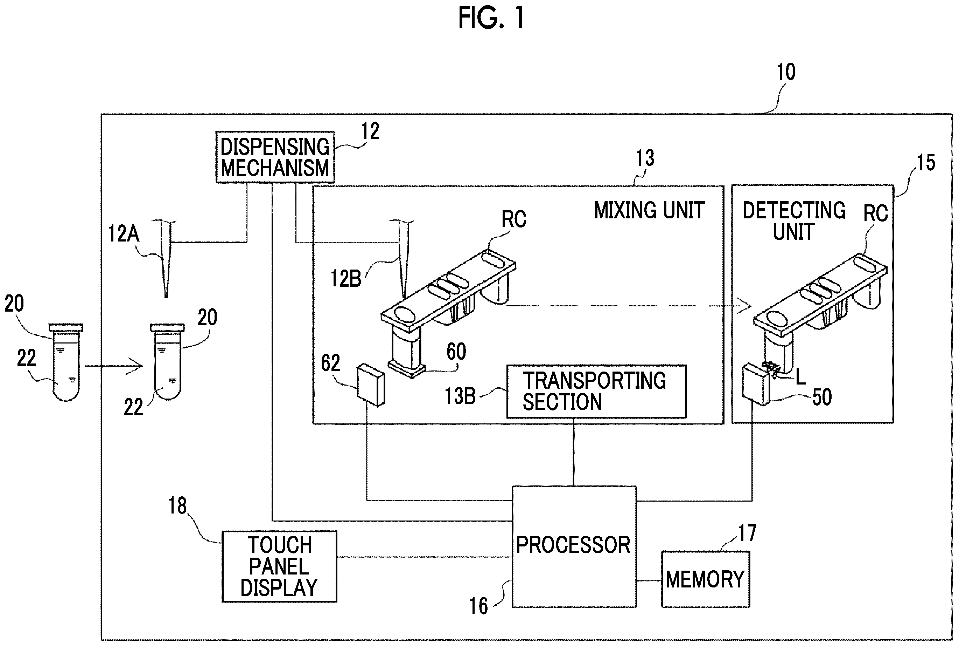

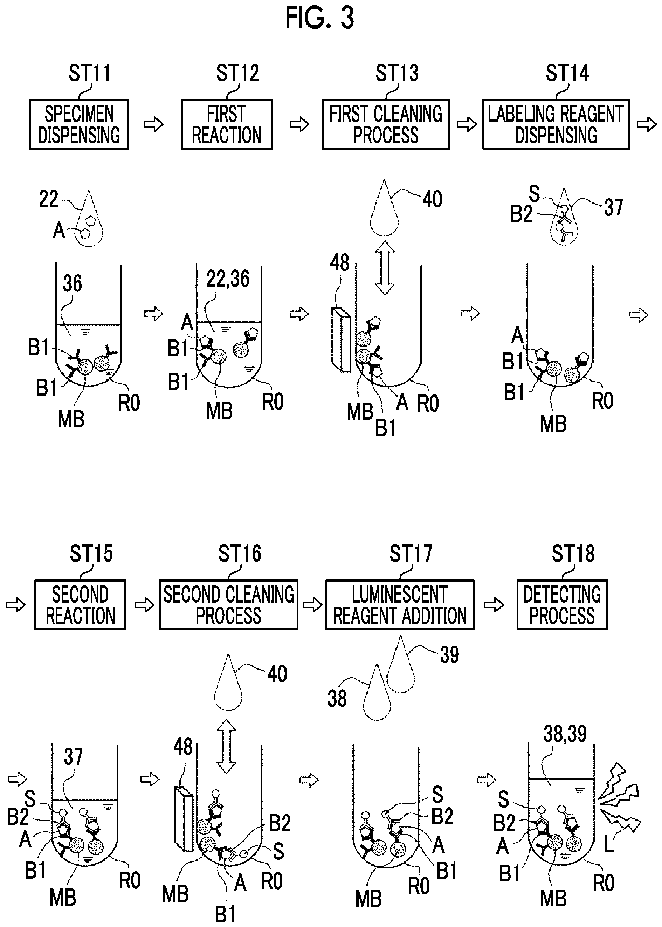

Fig. 1 is a schematic diagram showing an overall configuration of the examination apparatus 10 according to the embodiment of the present disclosure. As an example, the examination apparatus 10 is an immunological analysis apparatus which detects an examination target substance A in a specimen 22 collected from a biological body by an antigen-antibody reaction. The examination apparatus 10 optically detects the examination target substance A in the specimen 22 (see Fig. 3) using a cartridge RC having a reaction cell R0, and outputs an examination result.

As an example, the specimen 22 is a body fluid such as blood, collected from the biological body. In a case where the specimen 22 is blood, the specimen 22 may be whole blood, blood plasma, serum, or the like. In addition, a centrifuge may be provided in the examination apparatus 10 to extract blood plasma or serum from whole blood. Furthermore, the examination target substance A which can be contained in the specimen 22 is an antigen, an antibody, a protein, a low-molecular-weight compound, or the like. The specimen 22 is not limited to the blood, and may be a substance collected from the biological body, such as urine and body fluid. A specimen collection container 20 which accommodates the specimen 22 to be examined is loaded into the examination apparatus 10 and is subjected to examination. A plurality of the specimen collection containers 20 may be collectively loaded into the examination apparatus 10 such that the examination apparatus 10 can continuously process examinations for the plurality of the specimens 22.

The cartridge RC is attachably and detachably loaded into the examination apparatus 10. The cartridge RC is a single-use type used once for one specimen 22. As an example, the cartridge RC includes all reagent necessary for the examination of the specimen 22. In addition, a plurality of sets of the specimen collection container 20 and the cartridge RC may be collectively loaded into the examination apparatus 10 such that the examination apparatus 10 can continuously process examinations for the plurality of the specimens 22.

As an example, the examination apparatus 10 according to the present example performs an examination based on a chemiluminescent enzyme immunoassay method.

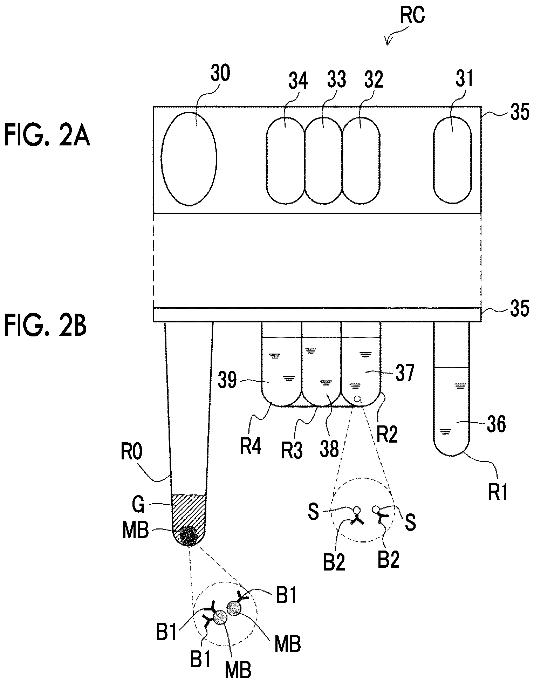

Figs. 2 are schematic views of an example of the cartridge RC, in which Fig. 2A is a top view of the cartridge RC and Fig. 2B is a front view of the cartridge RC. The cartridge RC includes a plate-shaped connection portion 35 having five openings 30 to 34, and five tubular cells R0 to R4 each having one end of each of the openings 30 to 34 and extending downward to include the reaction cell R0. The cartridge RC has a configuration in which the plurality of cells R0 to R4 are integrated by the connection portion 35. Among the plurality of cells R0 to R4, the reaction cell R0 and the cell R1 disposed at both ends are longer than the other cells R2 to R4. The reaction cell R0 is the longest. Before use, the openings 30 to 34 of the cartridge RC are covered with a sealing film (not shown).

The reaction cell R0 accommodates a plurality of magnetic particles MB modified with a first binding substance B1 for specifically binding to the examination target substance A, in a state of being sealed in gel-like gelatin G (see Fig. 2B). The reaction cell R0 is an example of the cell according to the technology of the present disclosure, which accommodates the reagent solidified with the gelatin G, and the magnetic particles MB modified with the first binding substance B1 is an example of the reagent according to the technology of the present disclosure. The "reagent solidified with the gelatin G" means a reagent in a state of being covered with the gelatin G and confined in the gelatin G The specimen 22 is dispensed into the reaction cell R0, and mixed with various reagents in the reaction cell R0. For example, in a case where the magnetic particles MB have a spherical shape, a diameter thereof is 0.1 to 10 µm, preferably 0.1 to 5 µm, and more preferably approximately 1 to 3 µm.

The cell R1 contains a buffer solution 36. The cell R2 accommodates a labeling reagent 37 containing the label S modified with a second binding substance B2 for specifically binding to the target substance. A first luminescent reagent 38 is contained in the cell R3, and a second luminescent reagent 39 is contained in the cell R4. In the present example, the label S is an enzyme, and the label S emits light in presence of the first luminescent reagent 38 and the second luminescent reagent 39. The buffer solution 36 in the cell R1, the labeling reagent 37 in the cell R2, the first luminescent reagent 38 in the cell R3, and the second luminescent reagent 39 in the cell R4 are simply referred to as reagents 36 to 39, in a case where it is not necessary to distinguish the respective liquids.

The first binding substance B1 and the second binding substance B2, which specifically bind to the target substance, are, for example, an antibody against an antigen in a case where the target substance is the antigen, an antigen against an antibody in a case where the target substance is the antibody, and an aptamer against a protein, a low-molecular-weight compound, or the like in a case where the target substance is the protein, the low-molecular-weight compound, or the like. The first binding substance B1 and the second binding substance B2 may be the same or different from each other.

Here, an examination procedure according to the present example based on the chemiluminescent enzyme immunoassay method will be described with reference to Fig. 3.

Fig. 3 schematically shows a reaction in a case where the specimen 22 contains the examination target substance A.

The reaction cell R0 contains the magnetic particles MB modified with the first binding substance B1 in advance, and a buffer solution 36 is dispensed into the reaction cell R0 before the dispensing of the specimen 22. In this state, the specimen 22 is dispensed into the reaction cell R0 (step ST11). A step of removing the gelatin G from the magnetic particles MB sealed and accommodated by the gelatin G in the reaction cell R0, which is performed before the examination procedure, will be described later.

In the reaction cell R0, the magnetic particles MB, the specimen 22, and the buffer solution 36 are mixed with each other, and as a first reaction, a binding reaction in which the examination target substance A in the specimen 22 and the first binding substance B1 are specifically bound to each other occurs (step ST12). In the first reaction, the examination target substance A in the specimen 22 binds to the first binding substance B1, so that the examination target substance A is captured by the magnetic particles MB through the first binding substance B1.

Next, a first cleaning process (that is, Bound/Free (B/F) separation) for removing unreacted components other than the examination target substance A, which are captured by the magnetic particles MB, is performed (step ST13). A magnet 48 is disposed close to an outside of the reaction cell R0, a reaction liquid after the first reaction is discharged in a state in which the magnetic particles MB are collected on an inner wall surface of the reaction cell R0, and a cleaning liquid 40 is dispensed into the reaction cell R0. In the step ST13 in Fig. 3, a bidirectional arrow in the up-down direction shown at the upper portion of the reaction cell R0 schematically indicates a state in which the cleaning liquid 40 is dispensed into the reaction cell R0 and discharged from the reaction cell R0. In the first cleaning process, the dispensing and the discharging of the cleaning liquid 40 are repeated a plurality of times. The cleaning liquid 40 is also discharged from the reaction cell R0 in a state in which the magnet 48 is disposed close to the outside of the reaction cell R0 and the magnetic particles MB are collected on the inner wall surface of the reaction cell R0.

After the first cleaning process, the labeling reagent 37 is dispensed into the reaction cell R0 (step ST14). The labeling reagent 37 contains a second binding substance B2 to which the label S is imparted, which is a binding substance for specifically binding to the examination target substance A.

In the reaction cell R0, as a second reaction, a binding reaction in which the examination target substance A captured by the magnetic particles MB and the second binding substance B2 are specifically bound to each other (step ST15). As a result, the label S is imparted to the examination target substance A through the second binding substance B2.

Next, a second cleaning process (B/F separation) for removing unreacted components other than the second binding substance B2 bound to the examination target substance A, which are captured by the magnetic particles MB in the labeling reagent 37, is performed (step ST16). The second cleaning process (step ST16) is performed in the same manner as the first cleaning process (step ST13).

Thereafter, the first luminescent reagent 38 and the second luminescent reagent 39 are added to the reaction cell R0 (step ST17). The label S is an enzyme, and causes a chemiluminescence L in the presence of the first luminescent reagent 38 and the second luminescent reagent 39, containing a luminescent substrate. The examination target substance A is detected by detecting the chemiluminescence L (step ST18). The examination procedure is as described above.

As an example, the examination apparatus 10 includes a dispensing mechanism 12, a mixing unit 13, a detecting unit 15, a processor 16, a memory 17, and a touch panel display 18.

In the detecting unit 15, a detection process of detecting the examination target substance A in the specimen 22 is executed. The detecting unit 15 includes a photodetector 50 such as a photomultiplier tube and a photodiode. The photodetector 50 is disposed to face the reaction cell R0, and detects the chemiluminescence L caused by the label S bound to the examination target substance A. In the present example, an enzyme is used as the label S to detect the chemiluminescence L generated by reacting with the luminescent substrate.

The processor 16 integrally controls each unit of the examination apparatus 10. An example of the processor 16 is a central processing unit (CPU) which performs various types of control by executing a program. The CPU functions as a control unit which controls each unit by executing a program.

In addition, the processor 16 acquires information on a light amount of the chemiluminescence L detected by the photodetector 50, and calculates a density of the examination target substance A based on the information on the light amount.

The memory 17 is an example of a memory connected to or built into the CPU as the processor 16. For example, the memory 17 stores a control program. In addition to the control program, the memory 17 stores setting information which is preset in order for the processor 16 to perform the various types of control.

In addition, the memory 17 stores information indicating a correspondence relationship between the light amount of the chemiluminescence L detected by the photodetector 50 and the amount of the examination target substance A. The correspondence relationship is stored, for example, as a calibration curve represented by a function. The correspondence relationship may be a format of a table. The processor 16 calculates the amount of the examination target substance A from, for example, the light amount of the chemiluminescence L acquired from the photodetector 50, and the calibration curve stored in the memory 17.

The touch panel display 18 receives, from a user, an operation instruction such as an instruction to start the examination. In addition, the touch panel display 18 displays information such as an examination result.

In the mixing unit 13, the mixing process to be performed on the specimen 22 for the detecting process is carried out. Specifically, the step ST11 to the step ST17 in the examination step described with reference to Fig. 3 is performed in the mixing unit 13. The mixing unit 13 includes a loading section (not shown) into which the cartridge RC having a plurality of cells including the reaction cell R0 is loaded, and a transporting section 13B. The transporting section 13B transports the cartridge RC to a location at which each process step in the mixing unit 13 is executed. Furthermore, the transporting section 13B transports the cartridge RC from the mixing unit 13 to the detecting unit 15.

In addition, the mixing unit 13 includes a heater 60 which heats the reaction cell R0 and a camera 62.

The heater 60 heats the reaction cell R0 of the cartridge RC loaded in the loading section to dissolve the gelatin G As an example, the heater 60 is disposed below the reaction cell R0. The heater 60 is an example of a heating unit. The gel-like gelatin G in the reaction cell R0 is dissolved and liquefied by being heated by the heater 60. The processor 16 controls the heater 60 to dissolve the gelatin G A preset heating condition set by the heater 60 (hereinafter, referred to as an initial heating condition) is stored in the memory 17. The initial heating condition includes, for example, at least a heating temperature of 36°C and a heating time of 1 minute. The dissolved gelatin G is suctioned and discarded by the dispensing mechanism 12. As a result, the gelatin G is removed from the reaction cell R0, and the magnetic particles MB are left and subjected to the examination.

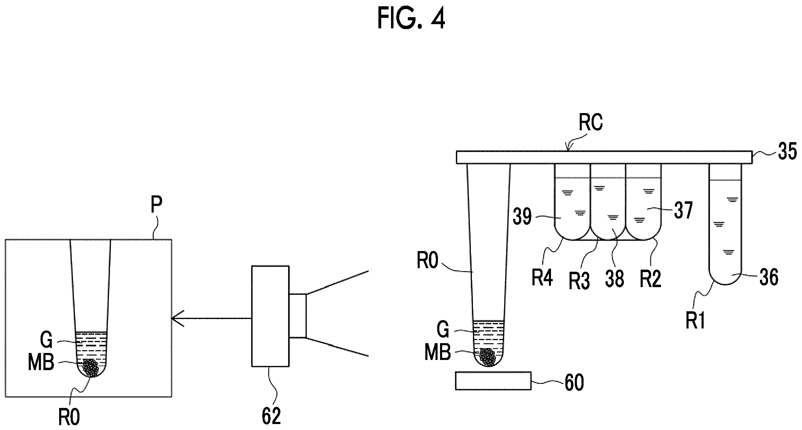

A shape of the heater 60 is not limited to the plate shape shown in Fig. 4, and may be, for example, a bottomed cylindrical shape, a square columnar shape, or the like, which can be inserted into at least a lower part of the reaction cell R0. In a case where the heater 60 has a shape which can be inserted into at least the lower part of the reaction cell R0 and is capable of heating not only from the bottom surface of the reaction cell R0 but also from the side surface, it is easy to prevent the undissolved gelatin G A heater having an appropriate shape may be used depending on the shape of the cartridge RC, the camera position, and the like.

The camera 62 is disposed to be capable of imaging a state of the gelatin G in the reaction cell R0 of the cartridge RC loaded in the mixing unit 13 (see Fig. 4). As shown in Fig. 4, the camera 62 images the state of the gelatin G in the reaction cell R0, and outputs the captured image P. The processor 16 determines the presence or absence of the undissolved gelatin G or the presence or absence of a sign of the undissolved gelatin G based on the image P. The camera 62 is an optical camera, and includes an image sensor and an optical system which forms an image of an object on an imaging plane. The image sensor is a complementary metal oxide semiconductor (CMOS) image sensor, a charge coupled device (CCD) image sensor, or the like.

The dispensing mechanism 12 is a mechanism which dispenses a liquid, and includes nozzles 12A and 12B, which suction and discharge the liquid, a moving mechanism (not shown), and a suction-discharge mechanism (not shown). The moving mechanism is a mechanism which three-dimensionally moves the nozzles 12A and 12B in a vertical direction (up-down direction). The suction-discharge mechanism is a mechanism which causes the nozzles 12A and 12B to perform a suction operation of suctioning the liquid and a discharge operation of discharging the liquid from the nozzles 12A and 12B. The moving mechanism includes an actuator such as a motor, which generates a driving force, and a driving force transmission mechanism consisting of a gear, a drive belt, and the like. The suction-discharge mechanism is composed of a pump or the like, which generates a driving force for the suction and discharge.

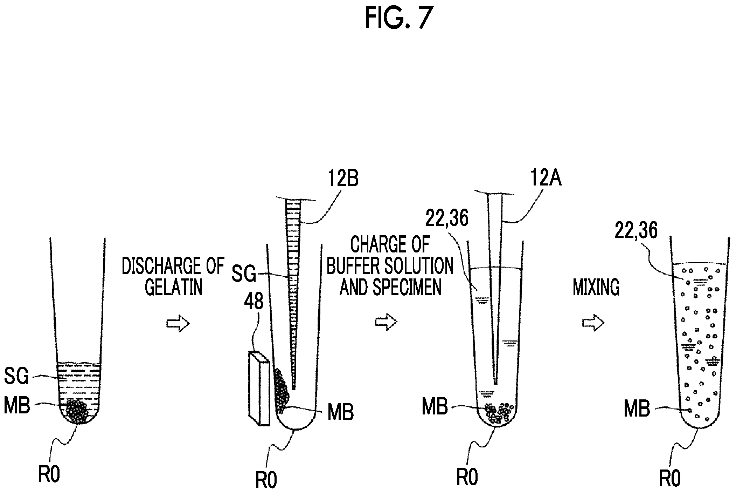

The dispensing mechanism 12 performs suction and dispensing of the liquid such as the specimen 22 and the reagents 36 to 39 using the nozzles 12A and 12B. Specifically, before the examination step described above is performed, the gelatin G dissolved in the reaction cell R0 is first suctioned (see Fig. 7). The magnet 48 (see Fig. 7) is positioned on the side wall of the reaction cell R0, and the gelatin G is suctioned in a state of being adsorbed on the wall surface by the magnet 48. Thereafter, suction and discharge of the reagent and the like are performed in the examination step.

In Fig. 1, for convenience, only the nozzle 12A for suctioning and discharging the specimen 22 and the nozzle 12B for suctioning the gelatin G are shown, but in reality, the dispensing mechanism 12 is provided with a nozzle for each liquid of each of the reagents 36 to 39 and the cleaning liquid 40.

As described above, the examination apparatus 10 includes the camera 62 which images the reaction cell R0. For example, the camera 62 images a state of the gelatin G in the reaction cell R0 after the reaction cell R0 is heated by the heater 60 to dissolve the gelatin G

The processor 16 analyzes the image P showing the state of the gelatin G acquired from the camera 62 to determine the presence or absence of undissolved gelatin G As an example, the processor 16 analyzes the image P, derives a dissolved state of the gelatin G after being dissolved, and determines the presence or absence of the undissolved gelatin G

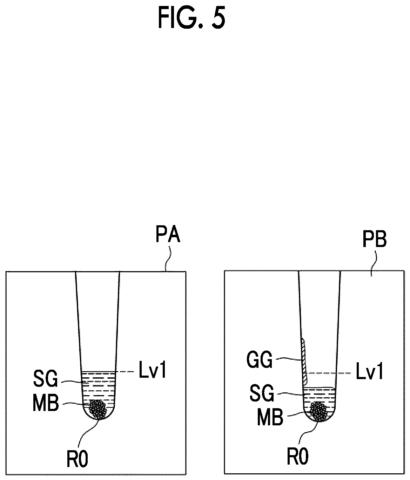

Fig. 5 shows a schematic diagram of images PA and PB, in which a state of the gelatin G after the dissolution is imaged. The state of the gelatin G shown in the image PA is a state in which the gelatin G is dissolved and all the gelatin G is uniformly liquefied to be a liquid gelatin SG The state of the gelatin G shown in the image PB is a state in which the gelatin G is undissolved and a part of the gelatin G is a gel-like gelatin GG without being liquefied and attached to the wall surface of the reaction cell R0. In Fig. 5, for the gelatin G, liquid gelatin is denoted by SG and gel-like gelatin is denoted by GG The same applies to Figs. 7 to 9 described later.

In the derivation of the dissolved state, the processor 16 detects, for example, whether or not the gelatin G is present on the wall surface of the reaction cell R0 above a liquid level position Lv1 in a case where all the gelatin G is uniformly liquefied. In a case where the gelatin G is present on the wall surface of the reaction cell R0 above the liquid level position Lv1 in a case where all the gelatin G is uniformly liquefied, the processor 16 determines that the undissolved matter is present. In addition, in a case where the gelatin G is not present on the wall surface of the reaction cell R0 above the liquid level position Lv1 in a case where all the gelatin G is uniformly liquefied, the processor 16 determines that there is no undissolved matter. The amount of the gelatin G is constant regardless of the cartridge RC, and it is sufficient that information on the liquid level position Lv1 in a case where all the gelatin G is uniformly liquefied is stored in the memory 17 as a setting information.

The determination of the presence or absence of the undissolved gelatin G in the processor 16 is not limited to the above description, and the determination may be performed by comparing the liquid level position Lv1 in a case where all the gelatin G is uniformly liquefied with a liquid level position of the liquid gelatin SG in the images PA and PB. That is, the processor 16 may determine that the undissolved matter is not present as the liquid level position Lv1 in a case where all the gelatin G is uniformly liquefied and the liquid level position of the liquid gelatin SG in the images PA and PB match each other; and may determine that the undissolved matter is present in a case where the liquid level position Lv1 and the liquid level position of the liquid gelatin SG in the images PA and PB do not match each other.

In addition, the processor 16 controls the heater 60 in a case where it is determined that the undissolved matter is present, to promote the dissolution of the gelatin G Specifically, the heating by the heater 60 is extended to promote the dissolution of the undissolved gelatin G In addition, in a case where the heating is extended, the temperature may be increased from the initial setting condition.

A heating condition in the additional heating can be appropriately set. An additional heating condition including a dissolving time and a dissolving temperature may be set according to, for example, the amount (volume) of the gelatin G adhering to the wall surface and the distance from the surface position. The area of the gelatin G adhering to the wall surface and the distance from the surface position in the image P are obtained by image analysis. The volume of the undissolved gelatin G can be estimated from the area of the gelatin G adhering to the wall surface in the image P. The distance is, for example, a distance between the surface position and a position farthest from the surface position in the gelatin G adhering to the wall surface. The temperature and time required for the dissolution may be determined, for example, by setting a longer dissolving time and/or a higher dissolving temperature as the volume is larger, and setting a longer dissolving time and/or a higher dissolving temperature as the distance from the surface position is longer.

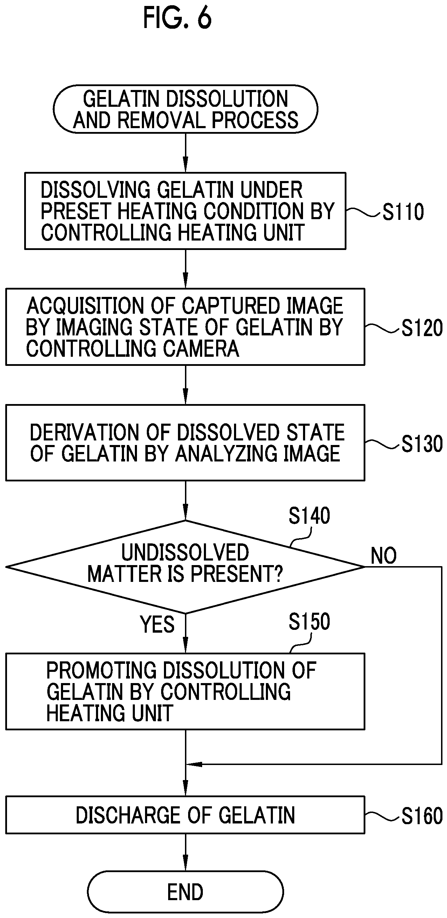

Hereinafter, operations of the above-described configuration will be described with reference to Fig. 6. Fig. 6 shows a first flow of a gelatin dissolution and removal process performed before the examination step shown in Fig. 3.

The processor 16 receives examination start instruction, and executes the heating of the reaction cell R0 of the cartridge RC by the heater 60. Here, the gelatin G is dissolved by heating by the heater 60 under a preset heating condition (step S110).

Next, the processor 16 dissolves the gelatin G under the preset heating condition, and then controls the camera 62 to image the state of the gelatin G in the reaction cell R0 and to acquire the image P in which the state of the gelatin G is imaged (step S120).

The processor 16 derives the dissolved state of the gelatin G by analyzing the acquired image P (step S130). As an example, as described above, in the derivation of the dissolved state, the processor 16 detects whether or not the gelatin G is present on the wall surface above a liquid level position Lv1 in a case where all the gelatin G is uniformly liquefied.

Next, the processor 16 determines whether or not the undissolved matter is present, that is, the presence or absence of the undissolved matter (step S140). In the image P, in a case where the gelatin G is detected on the wall surface above the liquid level position Lv1 in a case where all the gelatin G is uniformly liquefied, the processor 16 determines that the undissolved matter is present (step S140: YES). In addition, in a case where the gelatin G is not detected on the wall surface above the liquid level position Lv1, the processor 16 determines that there is no undissolved matter (step S140: NO).

The processor 16 controls the heater 60 in a case where it is determined that the undissolved matter is present (step S140: YES), to promote the dissolution of the gelatin G (step S150). For example, as described above, the heating by the heater 60 is extended to promote the dissolution of the undissolved gelatin G

The dissolved gelatin G is suctioned to remove the gelatin G from the reaction cell R0 (step S160), thereby ending the dissolution and removal process of the gelatin G

On the other hand, in a case where it is determined that the undissolved matter is not present (step S140: NO), since the gelatin G is sufficiently dissolved, with the processor 16, the dissolved gelatin G is suctioned to remove the gelatin G from the reaction cell R0 (step S160), thereby ending the dissolution and removal process of the gelatin G

As described above, the examination apparatus 10 which is an example of the embodiment includes the heater 60 (an example of the heating unit) which dissolves the gelatin G, the camera 62 which images the state of the gelatin G in the reaction cell R0 (an example of the cell), and the processor 16 which controls the heater 60 and the camera 62. The processor 16 acquires the image P showing the state of the gelatin G imaged by the camera 62 to determine the presence or absence of undissolved gelatin G As a result, it is possible to grasp the presence or absence of the undissolved gelatin G

In the above-described example, the processor 16 is configured to dissolve the gelatin G by the heater 60 under a preset heating condition, configured to image the state of the gelatin G by controlling the camera 62, configured to derive a dissolved state of the gelatin G after dissolving the gelatin G, and configured to determine the presence or absence of the undissolved gelatin G By performing the imaging after the dissolution, it is possible to reliably determine the presence or absence of undissolved matter.

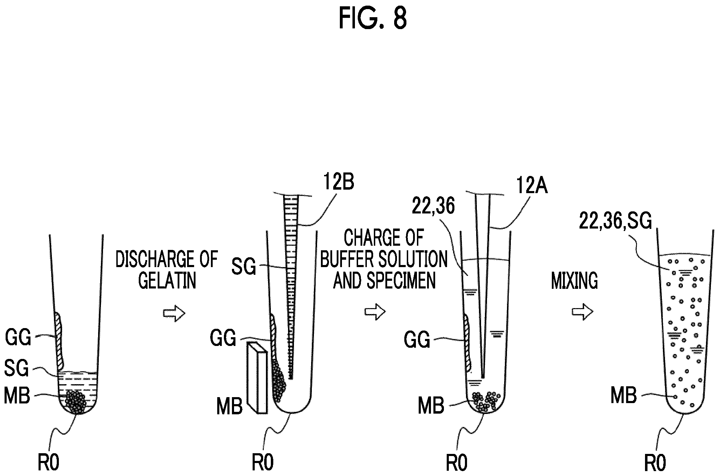

Here, a problem in a case where the gelatin G is undissolved will be described. Fig. 7 shows a step from suction of the gelatin G to the first reaction in a case where there is no undissolved matter, and Fig. 8 shows a step from suction of the gelatin G to the first reaction in a case where there is undissolved matter.

As shown in Fig. 7, the liquid gelatin SG is suctioned by the nozzle 12B. The liquid gelatin SG is suctioned by the nozzle 12B in a state in which the magnet 48 is disposed close to the outside of the reaction cell R0 and the magnetic particles MB are collected on the inner wall surface of the reaction cell R0 by the magnet 48. Since the gelatin G has no undissolved matter, substantially all of the gelatin G is suctioned by the nozzle 12B. Thereafter, the buffer solution 36 and the specimen 22 are put into the reaction cell R0 (see the step ST11 shown in Fig. 3). Furthermore, the first reaction (see the step ST12 shown in Fig. 3) is promoted by mixing the buffer solution 36, the specimen 22, and the magnetic particles MB by repeating the suction and discharge by the nozzle 12A.

Even in a case where the gelatin G is undissolved, as shown in Fig. 8, the liquid gelatin SG is suctioned by the nozzle 12B in the same manner as in the case of Fig. 7. The liquid gelatin SG is suctioned by the nozzle 12B, but the gel-like gelatin GG adhering to the wall surface is not suctioned and remains in the reaction cell R0. In this state, the buffer solution 36 and the specimen 22 are put into the reaction cell R0. Therefore, in a case of being mixed with the buffer solution 36, the specimen 22, and the magnetic particles MB, the gel-like gelatin GG is dissolved or mixed in the mixed liquid without being dissolved. In a case where the gelatin G which cannot be removed from the reaction cell R0 due to such poor solubility is mixed in the mixed liquid, the mixing with the specimen 22 and the magnetic particles MB which are the reagent is inhibited, that is, the first reaction is inhibited.

In the present examination apparatus 10, the processor 16 determines the presence or absence of the undissolved matter, and in a case where the undissolved matter is present, the gelatin dissolution is promoted, so that the undissolved matter can be reduced, and the gelatin G to be mixed in the mixed liquid in a case of mixing the specimen 22 and the reagent (here, the magnetic particles MB) can be reduced. As a result, an inhibitory factor against the reaction between the specimen 22 and the reagent can be suppressed, which leads to an improvement in reliability of the examination result.

In the above-described example, the gelatin G is dissolved and then the state of the gelatin G is imaged, but the state of the gelatin G may be imaged before the gelatin G is dissolved (that is, before the gelatin G is heated by the heating unit) or during the dissolution (that is, while the gelatin G is heated by the heating unit). That is, the processor 16 may be configured to acquire the image P in which the state of the gelatin G is imaged before the gelatin is dissolved or during the dissolution, which is imaged by the camera 62, and analyze the image P to determine the presence or absence of a sign of the undissolved gelatin G

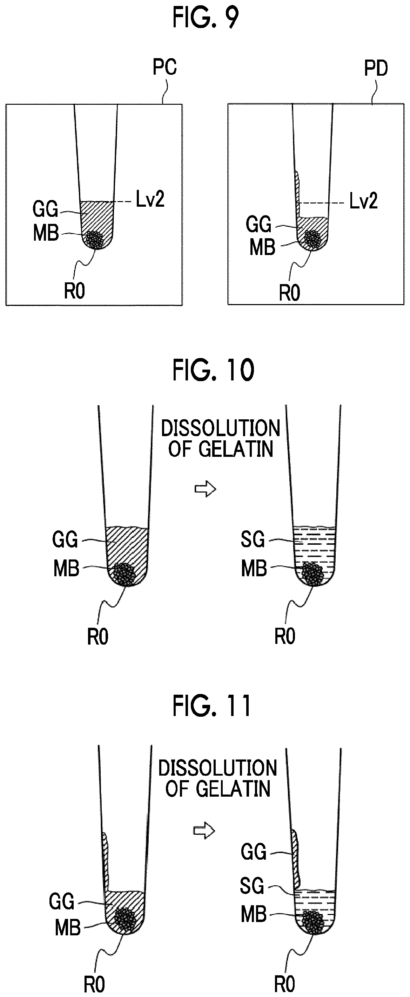

Fig. 9 shows a schematic diagram of images PC and PD, in which a state of the gelatin G before the dissolution is imaged. The state of the gelatin G shown in the image PC is a state in which the gelatin G is accommodated in the reaction cell R0 and the surface of the gel-like gelatin GG is substantially uniform. The state of the gelatin G shown in the image PD is a state in which the gelatin G is accommodated in the reaction cell R0 and a part of the gel-like gelatin GG is attached to the wall surface of the reaction cell R0 to be a non-uniform state.

As in the image PC, the heating condition (hereinafter, referred to as an initial heating condition) is set in advance to a temperature and a time at which the entire gelatin G is uniformly dissolved under a state in which the surface is in a uniform state before heating by the heater 60. The initial heating condition is stored in the memory 17. Therefore, as shown in Fig. 10, in a case where the surface of the gelatin G before the dissolution is in a uniform state, the gelatin G is heated under the initial heating condition to be uniformly dissolved as a whole to be the liquid gelatin SG, that is, there is no undissolved matter. Therefore, it can be said that the state of the gelatin G in the image PC of Fig. 9 is without any sign of the undissolved matter. In a case where there is no undissolved matter after the dissolution, as shown in Fig. 7, the gelatin G is suppressed from being mixed into the mixed liquid of the buffer solution 36, the specimen 22, and the magnetic particles MB.

On the other hand, as shown in Fig. 11, in a case where the surface of the gelatin G before the dissolution is in a non-uniform state, even in a case where the gelatin G is heated under the initial heating condition, the gel-like gelatin GG attached to the wall surface may not be dissolved and may remain, that is, insufficient dissolution may occur. Therefore, it can be said that the state of the gelatin G in the image PD of Fig. 9 is a sign of the undissolved matter. In a case where the undissolved matter is present after the dissolution, as shown in Fig. 8, the gelatin G is mixed into the mixed liquid of the buffer solution 36, the specimen 22, and the magnetic particles MB.

Therefore, in a case where the image PC shown in Fig. 9 is acquired, the processor 16 determines that there is no sign of the undissolved matter, and in a case where the image PD shown in Fig. 9 is acquired, the processor 16 determines that there is a sign of the undissolved matter.

More specifically, the processor 16 detects, for example, whether or not the gelatin G is adhered to the wall surface of the reaction cell R0 above a gelatin surface position Lv2 in a case where the gel-like gelatin GG is accommodated in a uniform state. In addition, in a case where the gel-like gelatin GG is present on the wall surface of the reaction cell R0 above the surface position Lv2 in a case where all the gel-like gelatin GG is accommodated in the reaction cell R0 in a uniform state, the processor 16 determines that there is a sign of undissolved matter. In addition, in a case where the gel-like gelatin GG is not present on the wall surface of the reaction cell R0 above the surface position Lv2 in a case where all the gel-like gelatin GG is accommodated in the reaction cell R0 in a uniform state, the processor 16 determines that there is no sign of undissolved matter. The amount of the gelatin G is constant regardless of the cartridge RC, and it is sufficient that information on the gelatin surface position Lv2 in a case where all the gelatin G is accommodated in a uniform state is stored in the memory 17 as a setting information.

The determination of the presence or absence of the sign of the undissolved gelatin G in the processor 16 is not limited to the above description, and the determination may be performed by comparing the surface position Lv2 in a state in which all the gel-like gelatin GG is accommodated in the reaction cell R0 in a uniform state with a surface position of the gel-like gelatin GG in the images PC and PD. That is, the processor 16 may determine that there is no sign of the undissolved matter as the surface position Lv2 in a case where all the gelatin G is accommodated in a uniform state and the surface position of the gel-like gelatin GG in the images PC and PD match each other; and may determine that there is no sign of the undissolved matter in a case where the surface position Lv2 and the surface position of the gel-like gelatin GG in the images PC and PD do not match each other.

In this way, in the examination apparatus 10, even in a case where the processor 16 acquires the image P obtained by imaging the state of the gelatin G before the dissolution and analyzes the image P to determine the presence or absence of a sign of the undissolved gelatin G, it is possible to grasp the presence or absence of the undissolved gelatin G

In addition, in this case, it is preferable that the processor 16 is configured to change the initial heating condition in a case where it is determined that there is a sign of the undissolved matter. Specifically, at least one of the following setting is changed: (1) the time is set to be longer than an initial setting time (that is, the dissolving time of the gelatin G is extended), or (2) the temperature is set to be higher than an initial setting temperature (that is, the dissolving temperature of the gelatin G is increased). The processor 16 controls the heater 60 to dissolve the gelatin G under the newly set heating condition.

In a case where the initial setting time is set to 1 minute, the change in dissolving time is, for example, a change to 2 minutes. In a case where the initial setting temperature is set to 30°C, the change in dissolving temperature is, for example, a change to 32°C or a change to 35°C. Both the dissolving time and the dissolving temperature may be changed at the same time. The heating condition including the dissolving time and the dissolving temperature may be set, for example, according to the amount (volume) of the gelatin G adhering to the wall surface and the distance from the surface position. The area of the gelatin G adhering to the wall surface and the distance from the surface position in the image P are obtained by image analysis. The volume of the undissolved gelatin G can be estimated from the area of the gelatin G adhering to the wall surface in the image P. The distance is, for example, a distance between the surface position and a position farthest from the surface position in the gelatin G adhering to the wall surface. The temperature and time required for the dissolution may be determined, for example, by setting a longer dissolving time and/or a higher dissolving temperature as the volume is larger, and setting a longer dissolving time and/or a higher dissolving temperature as the distance from the surface position is longer.

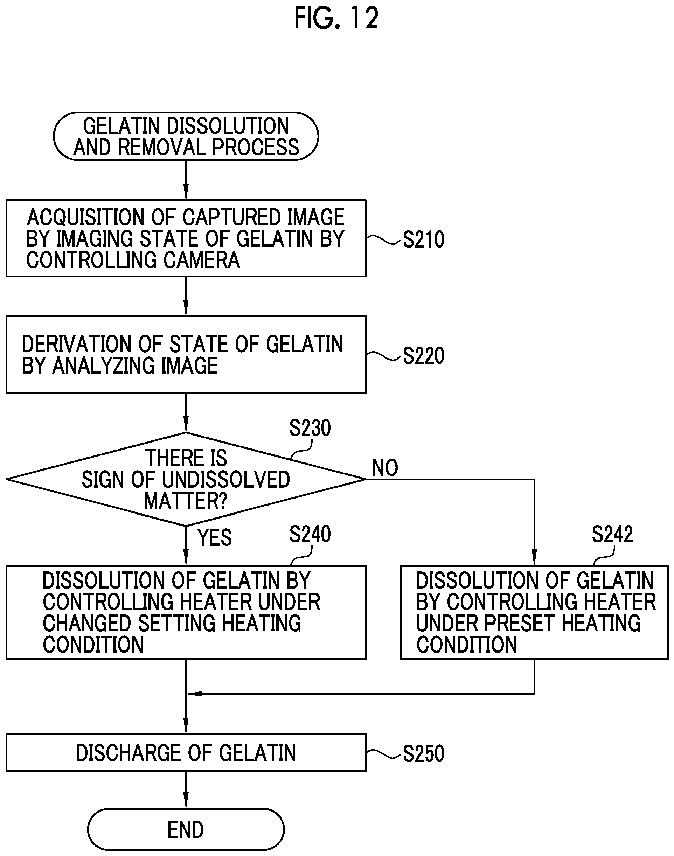

A second flow of the gelatin dissolution and removal process in a case where the image P of the state of the gelatin G is acquired before the dissolution of the gelatin G will be described with reference to Fig. 12.

The processor 16 receives examination start instruction, and first controls the camera 62 to image a state of the gelatin G in the reaction cell R0 and acquires the image P in which the state of the gelatin G is imaged (step S210).

The processor 16 derives the state of the gelatin G by analyzing the image P (step S220). As an example, as described above, in the derivation of the state of the gelatin G, the processor 16 detects whether or not the gelatin G is present on the wall surface above the surface position Lv2 in a case where all the gelatin G is uniformly accommodated in the reaction cell R0.

Next, the processor 16 determines the presence or absence of the sign of the undissolved matter (step S230). In the image P, in a case where the gelatin G is detected on the wall surface above the surface position Lv2 in a case where all the gelatin G is uniformly accommodated in the reaction cell R0, the processor 16 determines that there is a sign of the undissolved matter. In addition, in a case where the gelatin G is not detected on the wall surface above the surface position Lv2, the processor 16 determines that there is no sign of the undissolved matter.

On the other hand, in a case where it is determined that there is a sign of the undissolved matter (step S230: YES), the processor 16 changes the initial heating condition. Specifically, at least one of (1) changing the time to be longer than the initial setting time or (2) changing the temperature to be higher than the initial setting temperature is performed. The processor 16 controls the heater 60 to dissolve the gelatin G under the newly set heating condition (step S240).

The dissolved gelatin G is suctioned to remove the gelatin G from the reaction cell R0 (step S250), thereby ending the gelatin dissolution and removal process.

On the other hand, in a case where it is determined that there is no sign of the undissolved matter (step S230: NO), since the gelatin G is sufficiently dissolved under the initial heating condition, the processor 16 controls the heater 60 to heat the gelatin G under the preset heating condition for dissolving the gelatin G (step S242). The gelatin G is dissolved by heating under the initial heating condition without insolubilization. Thereafter, the dissolved gelatin G is suctioned to remove the gelatin G from the reaction cell R0 (step S250), thereby ending the gelatin dissolution and removal process.

In this way, in a case where the processor 16 determines the presence or absence of a sign of the undissolved matter, and in a case where there is a sign of the undissolved matter, the processor 16 changes the setting of the heating condition or the like to promote the dissolution of the gelatin G, so that the undissolved matter can be reduced. By reducing the undissolved gelatin G, the gelatin G to be mixed in the mixed liquid in a case of mixing the specimen 22 and the reagent (here, the magnetic particles MB) can be reduced. As a result, an inhibitory factor against the reaction between the specimen 22 and the reagent can be suppressed, which leads to an improvement in reliability of the examination result.

Furthermore, as a modification example of the examination apparatus 10, a notification unit (in the present example, the touch panel display 18) for notifying an error may be provided, and in a case where it is determined that the gelatin is undissolved or there is a sign of the undissolved gelatin, the processor 16 may be configured to notify the undissolved gelatin G or the sign of the undissolved gelatin G by the notification unit.

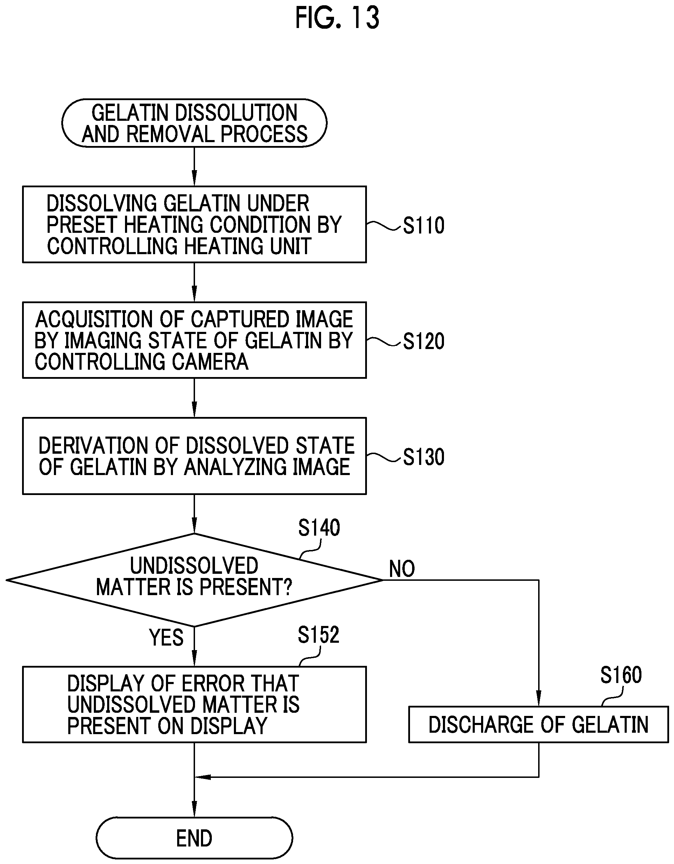

Fig. 13 shows a flow of a gelatin dissolution and removal process in a case where the notification unit is provided, as a third flow of the gelatin dissolution and removal process. In Fig. 13, the same steps as those of the first flow in Fig. 6 are denoted by the same reference numeral of step, and detailed description thereof will be omitted. In the third flow of the gelatin dissolution and removal process, in a case where it is determined that the gelatin G has the undissolved matter (step S140: YES), the processor 16 displays on the touch panel display 18 that the gelatin G has the undissolved matter (step S152). The processor 16 displays a message such as "gelatin G is not dissolved" on the touch panel display 18, for example. The touch panel display 18 is an example of the notification unit for notifying an error in the technology of the present disclosure. In addition, as the notification unit, a speaker which issues the notification of the error by voice, a light emitting unit which issues the notification of the error by emitting light, or the like may be provided separately from the touch panel display 18. In addition, instead of the message, the processor 16 may display the image P on the touch panel display 18 together with the message.

Similarly, even in the second flow, in a case where it is determined that there is a sign of the undissolved matter, an error display indicating that there is a sign of the undissolved gelatin G may be performed instead of changing the setting of the heating condition to dissolve the gelatin G

As described above, in the examination apparatus 10 of the modification example, the notification unit (in the present example, the touch panel display 18) for notifying an error is provided, and in a case where it is determined that the gelatin is undissolved or there is a sign of the undissolved gelatin, the processor 16 is configured to notify the undissolved gelatin G or the sign of the undissolved gelatin G by the notification unit. As a result, by the error, the user can be prompted to take a countermeasure to eliminate the undissolved gelatin G or to prevent the undissolved gelatin G The user can take measures such as changing the heating condition of the heater 60, or temporarily removing the cartridge RC from the examination apparatus 10 and dissolving the gelatin adhering to the wall surface outside the examination apparatus 10.

In addition, in a case where the image P is displayed on the touch panel display 18, the user can confirm the degree of the undissolved gelatin G or the degree of the sign of the undissolved matter. In this case, in a case where the degree of the undissolved gelatin G or the degree of the sign of the undissolved matter is not noticeable to the user, a countermeasure of continuing the measurement can also be performed.

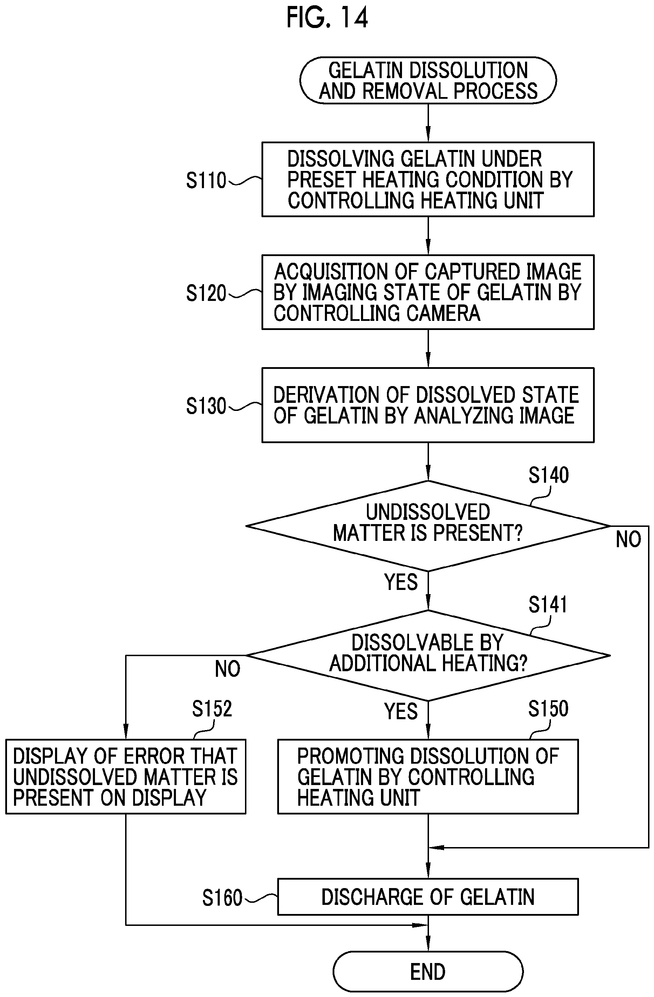

As a modification example of the third flow, Fig. 14 shows a fourth flow of the gelatin dissolution and removal process. In Fig. 14, the same steps as those of the third flow in Fig. 13 are denoted by the same reference numeral of step, and detailed description thereof will be omitted.

In the fourth flow, in a case where it is determined that the undissolved matter is present (step S140: YES), a step S141 of determining whether or not the undissolved matter can be dissolved by additional heating is provided. In the step 141, the processor 16 determines whether or not the gelatin G remaining in a gel form can be dissolved by performing the additional heating. For example, the heater 60 is disposed below the reaction cell R0 (see Fig. 4), and a part of the gelatin G is considered to be attached to the wall surface of the reaction cell R0 above the heater 60 such that the gelatin G cannot be dissolved by heating. For example, in the image analysis of the image P, the processor 16 determines that the gelatin G adhering to the wall surface cannot be dissolved by the additional heating, in a case where the distance from the surface position is equal to or more than a predetermined threshold value (step S141: NO). In a case where it is determined that the gelatin G cannot be dissolved by the additional heating (step S141: NO), the processor 16 performs an error display indicating that the undissolved matter is present on the touch panel display 18 (step S152), and ends the gelatin dissolution and removal process.

On the other hand, in a case where the processor 16 determines that the gelatin G is dissolvable by the additional heating (step S141: YES), the processor 16 controls the heater 60 to promote the dissolving of the gelatin G (step S150).

Similarly, even in the second flow, in a case where it is determined that there is a sign of the undissolved matter, it is determined whether or not the gelatin G is dissolvable by changing the setting of the heating condition; and in a case where it is determined that the undissolved matter is present even after changing the setting, an error display indicating that there is a sign of the undissolved gelatin G may be displayed, and the gelatin dissolution and removal process may be terminated.

As described above, in the examination apparatus 10, the notification unit (in the present example, the touch panel display 18) for notifying an error is provided, and in a case where it is determined that the gelatin is undissolved or there is a sign of the undissolved gelatin, the processor 16 may be configured to further determine whether or not the gelatin G can be dissolved by additional heating or heating by setting change. The processor 16 performs the additional heating or the heating by setting change in a case where it is determined that the gelatin G can be dissolved by the additional heating or the heating by setting change. On the other hand, in a case where it is determined that the gelatin G cannot be dissolved even by the additional heating or the heating by setting change, the processor 16 issues a notification that the undissolved gelatin G or a sign of the undissolved matter. As a result, in a case where the undissolved matter is present even with the additional heating or the heating by setting change, the user can be prompted to take a countermeasure for eliminating the undissolved gelatin G by the error. The user can take measures such as temporarily removing the cartridge RC from the examination apparatus 10 and dissolving the gelatin G adhering to the wall surface outside the examination apparatus 10.

As described above, according to the technology of the present disclosure, since the presence or absence of the undissolved gelatin G or the presence or absence of the sign of the undissolved matter can be grasped, the undissolved gelatin G can be eliminated or can be prevented. Therefore, it is possible to suppress the adverse effect on the examination result due to the remaining undissolved gelatin G, such as causing reaction inhibition in the subsequent step, and to improve the reliability of the examination result.

In the above-described examples, the immunological analysis apparatus which detects the examination target substance A contained in the specimen 22 by the antigen-antibody reaction has been described as an example of the examination apparatus 10. The technology of the present disclosure can be used for an examination apparatus other than the immunological analysis apparatus. In addition, the chemiluminescent enzyme immunoassay method has been described as an example of the method of detecting the examination target substance A, but the present disclosure is not limited to this method and may be applied to other methods.

In each of the above-described embodiments, as hardware structures of processing units that execute various types of process as the internal configuration of the processor 16, the following various processors can be used. The various processors include, for example, a CPU which is a general-purpose processor executing software to function as various processing units as described above, a programmable logic device (PLD), such as a field programmable gate array (FPGA), which is a processor whose circuit configuration can be changed after manufacture, or a dedicated electric circuit, such as an application specific integrated circuit (ASIC), which is a processor having a dedicated circuit configuration designed to perform a specific process.

One processing unit may be constituted by one of the various processors, or may be a combination of two or more processors of the same type or different types (for example, a combination of a plurality of FPGAs and/or a combination of a CPU and an FPGA). The plurality of processing units may be configured of one processor.

As an example of the plurality of processing units composed of one processor, first, as represented by computers such as a client and a server, there is a form in which one processor is composed of a combination of one or more CPUs and software and this processor functions as a plurality of processing units. A second example of the configuration is an aspect in which a processor that implements the functions of the whole system including a plurality of processing units using one integrated circuit (IC) chip is used, and a representative example of this aspect is a system-on-chip (SoC). As described above, the various processing units are configured using one or more of the various processors as the hardware structure.

More specifically, a circuitry combining circuit elements such as semiconductor elements may be used as the hardware structure of the various processors.

The present disclosure is not limited to the above-described embodiments, and can be implemented with appropriate modifications, such as omitting a configuration or replacing a configuration with a different configuration within the scope that does not deviate from the gist of the present disclosure.

The following appendixes are further disclosed with respect to the above embodiments.

### (Appendix 1)

An examination apparatus which examines a specimen using a cell accommodating a reagent solidified with gelatin, the examination apparatus including:
a heating unit for dissolving the gelatin;
a camera for imaging a state of the gelatin in the cell; and
a processor for controlling the heating unit and the camera,
in which the processor is configured to acquire an image of the state of the gelatin, which is imaged by the camera, and analyze the image to determine a presence or absence of undissolved gelatin or a presence or absence of a sign of undissolved gelatin.

### (Appendix 2)

The examination apparatus according to the appendix 1,
in which the processor is configured to dissolve the gelatin by the heating unit under a preset heating condition, configured to image the state of the gelatin by controlling the camera, configured to derive a dissolved state of the gelatin after dissolving the gelatin, and configured to determine the presence or absence of the undissolved gelatin.

### (Appendix 3)

The examination apparatus according to the appendix 1 or the appendix 2,
in which, in a case where it is determined that the gelatin is undissolved or there is a sign of the undissolved gelatin, the processor is configured to promote dissolution of the gelatin by controlling the heating unit.

### (Appendix 4)

The examination apparatus according to the appendix 3,
in which the processor is configured to perform at least one of control of extending a dissolving time of the gelatin or control of increasing a dissolving temperature of the gelatin with respect to the heating unit.

### (Appendix 5)

The examination apparatus according to any one of the appendixes 1 to 3, further including:
a notification unit for notifying an error,
in which, in a case where it is determined that the gelatin is undissolved or there is a sign of the undissolved gelatin, the processor is configured to notify the undissolved gelatin or the sign of the undissolved gelatin by the notification unit.

### (Appendix 6)

The examination apparatus according to any one of the appendixes 1 to 5,
in which an examination target substance in the specimen is detected by an antigen-antibody reaction.

### (Appendix 7)

The examination apparatus according to the appendix 6,
in which the reagent is magnetic particles modified with a binding substance specifically bound to the examination target substance.

The disclosure of Japanese Patent Application No. 2022-075196 filed on April 28, 2022 is incorporated in the present specification by reference. In addition, all documents, patent applications, and technical standards described in the present specification are incorporated herein by reference to the same extent as in a case of being specifically and individually noted that individual documents, patent applications, and technical standards are incorporated by reference.

## Claims

1. An examination apparatus which examines a specimen using a cell accommodating a reagent solidified with gelatin, the examination apparatus comprising:
a heating unit for dissolving the gelatin;
a camera for imaging a state of the gelatin in the cell; and
a processor for controlling the heating unit and the camera,
wherein the processor is configured to acquire an image of the state of the gelatin, which is imaged by the camera, and analyze the image to determine a presence or absence of undissolved gelatin or a presence or absence of a sign of undissolved gelatin.

2. The examination apparatus according to claim 1,
wherein the processor is configured to dissolve the gelatin by the heating unit under a preset heating condition, configured to image the state of the gelatin by controlling the camera, configured to derive a dissolved state of the gelatin after dissolving the gelatin, and configured to determine the presence or absence of the undissolved gelatin.

3. The examination apparatus according to claim 1,
wherein, in a case where it is determined that the gelatin is undissolved or there is a sign of the undissolved gelatin, the processor is configured to promote dissolution of the gelatin by controlling the heating unit.

4. The examination apparatus according to claim 3,
wherein the processor is configured to perform at least one of control of extending a dissolving time of the gelatin or control of increasing a dissolving temperature of the gelatin with respect to the heating unit.

5. The examination apparatus according to claim 1, further comprising:
a notification unit for notifying an error,
wherein, in a case where it is determined that the gelatin is undissolved or there is a sign of the undissolved gelatin, the processor is configured to notify the undissolved gelatin or the sign of the undissolved gelatin by the notification unit.

6. The examination apparatus according to any one of claims 1 to 5,
wherein an examination target substance in the specimen is detected by an antigen-antibody reaction.

7. The examination apparatus according to claim 6,
wherein the reagent is magnetic particles modified with a binding substance specifically bound to the examination target substance.
